# EUROPEAN PATENT APPLICATION

(11) **EP 4 810 003 A1**
(43) Date of publication of application: **23.09.2026**
(21) Application number: 23810272.7
(22) Date of filing: 16.11.2023
(51) Int. Cl.: A61K 8/06, A61K 8/9783, A61Q 19/08

(54) **CONCENTRATED COSMETIC COMPOSITION, DILUTED COSMETIC COMPOSITION, USE OF AND METHOD FOR PREPARING A DILUTED COSMETIC COMPOSITION**

(71) Applicant: Natura Cosméticos S.A., 05106-000 São Paulo - SP (BR)
(72) Inventor: DE SOUZA FERREIRA GARCIA, Cinthia Fernanda, 05106-000 São Paulo - SP (BR); DE PAIVA MORAIS, Bruno, 05106-000 São Paulo - SP (BR); FIGUERO GOMES, Leticia, 05106-000 São Paulo - SP (BR)
(74) Representative: Simmons & Simmons
(86) International application number: PCT/BR2023/050395
(87) International publication number: WO 2025/102128

(57) **Abstract**

The present invention deals with a concentrated cosmetic composition that is substantially free of water, comprising an emulsifying system and an emollient system which, when mixed in suitable amounts of water instantly provides an emulsion, having good stability against temperature and moisture variations and having good microbiological resistance. A diluted composition, use and method for preparing a diluted cosmetic composition are additional objects of the present invention.

## Description

### FIELD OF THE INVENTION

The present invention deals with a concentrated cosmetic composition that is substantially free of water, comprising an emulsifying system and an emollient system which, when mixed in suitable amounts of water instantly provides an emulsion, having good stability against temperature and moisture variations and having good microbiological resistance. A diluted composition, use and method for preparing a diluted cosmetic composition are also objects of the present invention.

### BACKGROUND OF THE INVENTION

In the cosmetic field water is in general the main component of cosmetic formulations, generally making up to around 50 to 90% of the total composition. It performs a role as a solvent, which makes some ingredients, compounds and substances capable of coming into contact with the skin safely and continuously. Furthermore, water is used in creams, gels, makeup compositions and other beauty and hygiene cosmetics, which demonstrates its cleaning properties.

However, despite its benefits, water is the main issue that leads to poor preservation of cosmetic products. Due to the large amount of water molecules, the formulations end up more exposed to microorganisms, such as fungi, which proliferate in highly moist environments. Therefore, preservatives are required in considerable amounts.

Furthermore, water has become an increasingly precious and scarce resource.

Therefore, water, although very useful in the manufacture of numerous cosmetic formulations, needs to have its use reconsidered.

Therefore, concentrated cosmetic formulations have been researched. In general, such compositions seek to increase the concentration of active ingredients, enhancing the product's action to offer faster results. Alternative carriers have also been researched.

Furthermore, reduced packaging sizes related to the reduction in the content of concentrated products, decrease the environmental impacts related to transportation and less material used to store the formulation.

For example, international application WO11140341 by L'Oreal, for example, describes an anhydrous mask composition comprising an acrylic thickener and an oil.

International application WO09000486 by Beiersdorf AG describes a cosmetic preparation containing: a) a hydroxyethyl acrylate/sodium acryloyldimethyl taurate copolymer in a concentration of from 0.1 to 3 percent, b) one or two water-soluble lipids in a total concentration of from 0.01 to 20 weight percent of the total weight of the preparation.

KR1020468021 by Amorepacific Corp., describes a solid cosmetic makeup composition of hydroxyethyl acrylate/sodium acryloyldimethyl taurate copolymer by emulsifying the aqueous fraction into an oily fraction.

As with most water-free cosmetic compositions, the international application in question discloses a composition that is intended to concentrate the active ingredients, but which does not address the water issue in order to achieve a composition that can be diluted for use with guaranteed stability.

Thus, there remains a need in the art for new cosmetic compositions containing low amounts of water, while being capable of being diluted, providing more sustainable products while maintaining efficiency and, mainly, stability over time, especially after dilution.

### SUMMARY OF THE INVENTION

In a first aspect, the present invention refers to a concentrated cosmetic composition that is substantially free of water, which comprises an emulsifying system and an emollient system which, when mixed in suitable amounts of water instantly provides an emulsion having good stability against variations in temperature and moisture and good microbiological resistance.

In a second aspect, the present invention contemplates a diluted composition, which comprises the concentrated cosmetic composition according to the present invention diluted in water in a base:water ratio of about 1:5 to about 1:10, preferably about 1:8 base:water.

In a third aspect, the present invention contemplates the use of the concentrated cosmetic composition in the preparation of a ready-to-use diluted cosmetic composition.

In a fourth aspect, the present invention relates to a method for the preparation of a diluted or transformed cosmetic composition comprising diluting a concentrated cosmetic composition in a base:water ratio of about 1:5 to 1:10, preferably about 1:8 base: water, under manual shaking or in a mixer.

### BRIEF DESCRIPTION OF THE FIGURES

Figure 1 shows the results in the reduction of skin dryness using the composition according to the present invention.
Figure 2 shows the results of the increase in the percentage of hydration from the application of the composition according to the present invention.

### DESCRIPTION OF THE INVENTION

The present invention deals with a concentrated cosmetic composition that is substantially free of water, comprising an emulsifying system and an emollient system which, when mixed in suitable amounts of water instantly provides an emulsion, having good stability against temperature and moisture variations and having good microbiological resistance.

The concentrated cosmetic composition according to the present invention allows the preparation of a "do-it-yourself" emulsion, that is, the combination of an emulsifying system with a specifically developed emollient system provides the possibility for the end user to prepare the emulsion for use by adding proper amounts of water under manual shaking or with the aid of a mixer.

The emulsion formed from the concentrated cosmetic composition and water forms instantly and has good stability against variations in temperature and moisture and has good microbiological resistance.

The diluted composition according to the present invention may further comprise cosmetically suitable excipients, such as preservatives, fragrances, dyes, pigments, active ingredients, among others known in the art.

The diluted cosmetic composition according to the present invention can be obtained by simple dilution with water in specific ratios, so as to maintain its features of stability, microbiological resistance and, particularly, cosmetic performance, for example, hydration.

In one embodiment, the concentrated cosmetic composition according to the present invention comprises:
(a) an emulsifying system consisting of at least one of hydroxyethyl acrylate/sodium acryloyldimethyl taurate copolymer, polysorbate 20, glyceryl oleate citrate, or mixtures thereof; and
(b) an emollient system consisting of at least one of *Bertholletia excelsa* seed oil, canola oil, isopropyl palmitate or mixtures thereof;
(c) cosmetically acceptable excipients.

The concentrated cosmetic composition according to the present invention is substantially free of water. By substantially water-free is meant that the composition may contain up to 5% water (moisture inherent to the ingredients), being preferably completely free of water.

In a particular embodiment, the cosmetic composition according to the present invention has a base comprising:
(a) 4 to 45% emulsifying system; and
(b) 2 to 70% emollient system;
(c) cosmetically acceptable excipients.

In another embodiment, the diluted cosmetic composition according to the present invention comprises:
(a) an emulsifying system consisting of at least one of hydroxyethyl acrylate/sodium acryloyldimethyl taurate copolymer, polysorbate 20, glyceryl oleate citrate, or mixtures thereof; and
(b) an emollient system consisting of at least one of *Bertholletia excelsa* seed oil, canola oil, isopropyl palmitate or mixtures thereof;
(c) cosmetically acceptable excipients;
wherein said composition comprises water in a base:water ratio of from 1:5 to 1:10, preferably about 1:8 base:water.

In a preferred embodiment, 28.8g of the concentrated cosmetic composition according to the present invention are used to 212.2g of water. Changes in these ratios will confer different texture results.

The compositions according to the present invention may be available in the form of an emulsion, tablet or soap, liquid, solid or semi-solid.

In another embodiment, the present invention further relates to the use of the concentrated cosmetic composition according to the present invention in the preparation of a ready-to-use diluted cosmetic composition. This preparation can be made by the end user using manual shaking or with the aid of any mixers known in the art.

In yet another embodiment, the present invention contemplates a method for preparing a diluted cosmetic composition comprising diluting a concentrated cosmetic composition in the ratios described herein under manual shaking or in a mixer.

The following examples, without being limited thereby, show the particular embodiments of the present invention and demonstrate that the instant combination of ingredients has been developed to provide the skin with an anti-drying action, which is demonstrated by corneometry tests and clinical assessment, providing hydration for up to 72 hours. These results show that the composition according to the present invention is efficient to replenish the skin, leaving the skin nourished, as evidenced by corneometry and barrier fortification tests, improves skin texture as demonstrated via image analysis in addition to bringing sensory benefits as demonstrated by trained panel and sensory research, such as velvety effect, rapid absorption, among others.

### EXAMPLES

### Example 1 - Concentrated cosmetic composition according to the present invention

**Table 1A - Concentrated cosmetic composition according to the present invention**

| Ingredient | Concentration (%) |
|---|---|
| *Bertholletia excelsa* seed oil | 25.08 |
| Canola oil | 21.41 |
| Fragrance | 10 |
| Polysorbate 20 | 8.33 |
| Isopropyl palmitate | 7.2 |
| Glyceryl oleate citrate | 6.664 |
| Caprylic/capric glycerides | 1.666 |
| Linalool | 0.8532 |
| Disodium EDTA | 0.84 |
| Polyglyceryl-3 caprylate | 0.83 |
| Benzyl Salicylate | 0.6901 |
| Potassium sorbate | 0.6 |
| Dehydroacetic acid | 0.6 |
| Sodium benzoate | 0.5 |
| Pentaerythrityl tetra-di-t-Butyl hydroxyhydrocinnamate | 0.42 |
| Limonene | 0.3759 |
| Coumarin | 0.138 |
| Alpha-isomethyl ionone | 0.1132 |
| Eugenol | 0.0347 |
| Citral | 0.0305 |
| Hydroxycitronellal | 0.022 |
| Benzyl benzoate | 0.019 |
| Geraniol | 0.0058 |

**Table 1B - Concentrated cosmetic composition according to the present invention**

| Ingredient | Concentration (%) |
|---|---|
| *Bertholletia excelsa* seed oil | 25.08 |
| Canola oil | 21.41 |
| Hydroxyethyl acrylate/sodium acryloyldimethyl taurate copolymer | 15.55 |
| Perfume | 9.16 |
| Polysorbate 20 | 8.33 |
| Isopropyl palmitate | 7.20 |
| Glyceryloleate citrate | 6.66 |
| Caprylic/capric glycerides | 1.67 |
| Sorbitan isostearate | 1.15 |
| Disodium EDTA | 0.84 |
| Triethyl citrate | 0.83 |
| Potassium sorbate | 0.60 |
| Dehydroacetic acid | 0.60 |
| Sodium benzoate | 0.50 |
| Pentaerythrityl tetra-di-t-butyl hydroxyhydrocinnamate | 0.42 |

### Example 2 - Diluted cosmetic composition according to the present invention

**Table 2A - Diluted cosmetic composition according to the present invention**

| Ingredient | Concentration (%) |
|---|---|
| Water | 88 |
| *Bertholletia excelsa* seed oil | 5.0136 |
| Canola oil | 2.4684 |
| Fragrance | 1.2 |
| Polysorbate 20 | 0.9996 |
| Isopropyl palmitate | 0.864 |
| Glyceryloleate citrate | 0.79968 |
| Caprylic/capric glycerides | 0.19992 |
| Linalool | 0.102384 |
| Disodium EDTA | 0.1008 |
| Polyglyceryl-3 caprylate | 0.0996 |
| Benzyl Salicylate | 0.082812 |
| Potassium sorbate | 0.072 |
| Dehydroacetic acid | 0.072 |
| Sodium benzoate | 0.06 |
| Pentaerythrityl tetra-di-t-butyl hydroxyhydrocinnamate | 0.0504 |
| Limonene | 0.045108 |
| Coumarin | 0.01656 |
| Alpha-isomethyl ionone | 0.013584 |
| Eugenol | 0.004164 |
| Citral | 0.00366 |
| Hydroxycitronellal | 0.00264 |
| Benzyl benzoate | 0.00228 |

**Table 2B - Diluted cosmetic composition according to the present invention**

| Ingredient | Concentration (%) |
|---|---|
| Water | 88.00 |
| *Bertholletia excelsa* seed oil | 3.01 |
| Canola oil | 2.57 |
| Hydroxyethyl acrylate/sodium acryloyldimethyl taurate copolymer | 1.87 |
| Sorbitan isostearate | 0.14 |
| Perfume | 1.10 |
| Polysorbate 20 | 1.00 |
| Glyceryloleate citrate | 0.80 |
| Caprylic/capric glycerides | 0.20 |
| Isopropyl palmitate | 0.86 |
| Disodium EDTA | 0.10 |
| Triethyl citrate | 0.10 |
| Potassium sorbate | 0.07 |
| Dehydroacetic acid | 0.07 |
| Sodium benzoate | 0.06 |
| Pentaerythrityl tetra-di-t-butyl hydroxyhydrocinnamate | 0.05 |

The compositions of example 2 were prepared from the compositions of example 1 by a "do it yourself" method, that is, by mixing the composition of example 1 with water in suitable ratios, 28.8g of the composition to 212.2g of water under manual shaking until a composition is instantly ready for use.

### Example 3 - Assessment of the effectiveness of a cosmetic product in reducing dry skin through subjective analysis of clinical effectiveness.

The aim of the present study was to assess the effectiveness of the investigational product in reducing skin dryness through a subjective clinical efficacy analysis.

When participants were recruited, they were instructed to stop using any cosmetic products on their legs 48 hours prior to the beginning of the study. On the first day of the study, participants who attended the laboratory received explanations from the researcher about the study procedures, ethical and legal aspects, risks and benefits, medical support and forms of reimbursement for participation expenses.

The reduction in skin dryness was assessed by a subjective analysis of clinical efficacy in the beginning of the study and 15 minutes, 4 hours and 8 hours after application of the experimental product.

According to the study protocol and the procedures used to assess effectiveness of the product under investigation in reducing skin dryness, it was possible to observe that:
- The product under investigation provided a significant reduction in skin dryness after 15 minutes, 4 and 8 hours after applying the product under investigation as compared to the control (skin with no products applied).
- The product under investigation reduced the intensity of skin dryness by 22.7% after 15 minutes and 21.9% after 4 and 8 hours after applying the product under investigation.
- 100% of research participants showed improvement in skin dryness after applying the product under investigation.

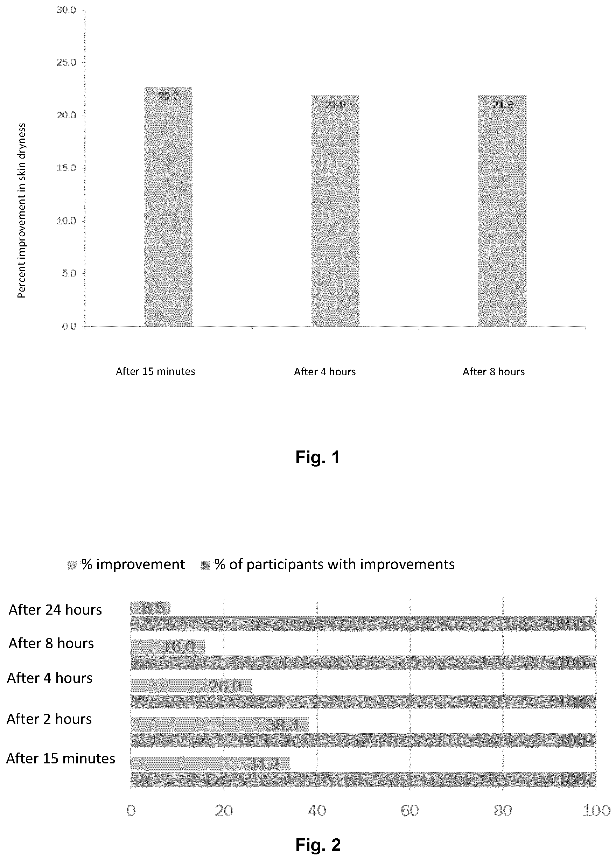

Figure 1 shows the results in the reduction of skin dryness.

### Example 4 - Assessment of the strengthening of the skin barrier provided by the use of a cosmetic product

The aim of this study was to assess the strengthening of the skin barrier after 14 and 28 days of home use of the product under investigation.

When participants were recruited, they were instructed to refrain from using any cosmetic products on their forearms 48 hours prior to the beginning of the study. On the day of the study, the subjects received explanations from the researcher about the study procedures, ethical and legal aspects, risks and benefits, medical support and forms of reimbursement for participation expenses.

The methodology included the assessment of transepidermal water loss after partial removal of the stratum corneum at the beginning of the study and after 14 and 28 days of use of the product under investigation. To assess the skin barrier strengthening effect, the tape stripping procedure was carried out by repeatedly (30 times) applying and removing a transparent medical tape (Transpore 3M, 3M, Brazil) at marked locations on the volar forearms, followed by measurement of transepidermal water loss (Tewameter^{®} TM HEX and Multiprobe Adapter MPA, CKeletronics, Germany).

**Table 3 - Barrier strengthening results.**

| Assessment time | % improvement in strengthening of the skin barrier | % of research subjects who showed an improvement |
|---|---|---|
| After 14 days | 9.3% | 100% |
| After 28 days | 14.5% | 100% |

According to the study protocol and procedures used to assess the strengthening effect on the skin barrier provided by the experimental product, it was possible to note that:
- The product under investigation provided a significant effect of strengthening the skin barrier after 14 and 28 days of home use of the product under investigation as compared to the control (untreated skin).
- The product under investigation provided a significant skin barrier strengthening effect of 9.3% after 14 days and 14.5% after 28 days relative to the initial skin condition and the control.
- 100% of the research subjects showed strengthening of the skin barrier after 14 and 28 days of home use of the product under investigation.

### Example 5 - Assessment of skin roughness after treatment with the product under investigation

19 research subjects were included and completed the study; Average ages: 49 ± 10 years old. Phototype (Fitzpatrick): 84% phototype III, 11% phototype IV and 5% phototype V.

When recruiting research participants, they were instructed to stop using any cosmetic products on their legs 48 hours prior to the beginning of the study.

On the day of the study, the subjects received explanations from the researcher about the study procedures, ethical and legal aspects, risks and benefits, medical support and forms of reimbursement for participation expenses.

The methodology consisted of assessing the improvement in skin roughness through image analysis. Photographs of the legs were taken in the beginning of the study and after 30 minutes of application of the product under investigation.

After 30 minutes of application of the experimental product, it was observed that: (1) a 24.8% reduction reaching up to 34.8% in skin roughness and (2) 84% of research subjects showed a reduction in skin roughness.

According to the obtained results, treatment with the product under investigation showed the necessary efficacy to support the following statement: Improves skin roughness after 30 minutes of application of the product under investigation.

### Example 5 - Assessment of the improvement in skin hydration provided by applying the product under investigation.

When participants were recruited, they were instructed to refrain from using any cosmetic products on their forearms 48 hours prior to the beginning of the study.

On the first day of the study, participants who attended the laboratory received explanations from the researcher about the study procedures, ethical and legal aspects, risks and benefits, medical support and forms of reimbursement for participation expenses.

Participants remained in an air-conditioned room with a temperature of 22°C ± 2 and 55% ± 5 relative humidity for 30 minutes.

After the stabilization period, baseline capacitance measurements were obtained. Then, the experimental product was applied, and the participants remained in the laboratory for measurements after 15 minutes, 2, 4 and 8 hours.

In the next days, participants returned to the laboratory for 24-, 48- and 72-hour measurements after applying the product under investigation.

Figure 2 shows the results of the increase in the percentage of hydration from the application of the composition according to the present invention.

According to the study protocol and the procedures used to assess effectiveness of the product under investigation in increasing skin hydration, it was possible to observe that:
- The product under investigation provided a significant increase in skin hydration after 15 minutes, 2, 8 and 24 hours of application relative to the control (skin with no products applied) and the baseline condition;
- The experimental product retained skin hydration for up to 24 hours after application;
- The experimental product increased the skin hydration level by up to 38.2%. 100% of participants showed an improvement in skin hydration after applying the product under investigation.

### Example 6 - Skincare product panel

The composition according to the present invention was assessed descriptively using the emulsion "Perception" trained panel.

In the test, each of the assessed parameters (except for the parameter of absorption point) was quantified on a 10 cm unstructured linear scale anchored with little/medium/a lot according to the quantitative references for each parameter. For the parameter of absorption point, the number of rotations required for the product to begin to be absorbed by the skin was recorded.

Assessment was carried out in sensory booths with 13 trained and validated examiners.

Temperature and moisture of the sensory booths were monitored throughout the study and maintained comfortable for the examiners.

The results were statistically assessed using XLSTAT 2023 and FIZZ 2.46B software.

As a result, the tested composition was shown to present good performance in general, with the following noticeable parameters: dry touch, spreadability and sliding.

The tests carried out showed that the moisturizer formed according to the present invention exhibited good stability against temperature and moisture changes and good microbiological resistance.

Furthermore, the ingredients were combined in order to provide an anti-drying action, as can be seen in corneometry and clinical assessment tests, hydrating the skin for up to 72 hours. Furthermore, it was efficient in replenishing the skin, leaving it nourished, as evidenced by corneometry and barrier fortification tests, improving its texture (as demonstrated via image analysis), in addition to providing sensory benefits proven via a trained panel, such as a velvety effect, rapid absorption, among others.

### Example 6 - Stability study

The accelerated stability study of cosmetic products is intended to determine the period of time from the date of manufacture in which the product will retain its physical-chemical and sensory features within acceptable standards through exposure to different temperature and moisture conditions.

The composition according to the present invention was assessed taking into account parameters of application, appearance, color, smell, viscosity, interaction with the packaging, pH, among others.

Based on the results achieved for one and three months of accelerated stability studies, the composition according to the present invention retained its features within acceptable standards, therefore being considered approved, with an estimated shelf life of 730 days (24 months).

### Example 7 - Preservative efficacy assessment

The composition according to the present invention was assessed against a group of gram positive bacteria: *Staphylococcus aureus* ATCC 6538, *Staphylococcus epidermidis* ATCC 12228.

It was also assessed in view of a group of gram negative bacteria: *Escherichia coli* ATCC 8739, *Enterobacter cloacae* ATCC 13047 *Pseudomonas aeruginosa* ATCC 9027, *Burkholderia cepacia* ATCC 25416.

Finally, it was assessed in view of a group of fungi: *Candida albicans* ATC 10231, *Aspergillus brasiliensis* ATCC 16404.

The composition according to the present invention was deemed approved.

It was also noted that the composition according to the present invention remains suitable for use after dilution with regard to stability.

The person skilled in the art will be able to readily assess through the teachings of the instant text and examples the advantages of the invention and to propose variations and equivalent alternatives of implementation without departing from the scope of the invention as defined in the appended claims.

## Claims

1. A CONCENTRATED COSMETIC COMPOSITION **characterized in that** it has a base that comprises:
(a) an emulsifying system consisting of at least one of hydroxyethyl acrylate/sodium acryloyldimethyl taurate copolymer, polysorbate 20, glyceryl oleate citrate, or mixtures thereof; and
(b) an emollient system consisting of at least one of *Bertholletia excelsa* seed oil, canola oil, isopropyl palmitate or mixtures thereof;
(c) cosmetically acceptable excipients;
wherein said composition is substantially free of water.

2. The COMPOSITION according to claim 1, **characterized in that** it comprises:
(a) 4 to 45% emulsifying system; and
(b) 2 to 70% emollient system;
(c) cosmetically acceptable excipients;

3. The COMPOSITION according to claim 1, **characterized in that** it comprises up to 5% water.

4. A DILUTED COSMETIC COMPOSITION **characterized in that** it has a base consisting of:
(a) an emulsifying system consisting of at least one of hydroxyethyl acrylate/sodium acryloyldimethyl taurate copolymer, polysorbate 20, glyceryl oleate citrate, or mixtures thereof; and
(b) an emollient system consisting of at least one of *Bertholletia excelsa* seed oil, canola oil, isopropyl palmitate or mixtures thereof;
(c) cosmetically acceptable excipients;
wherein said composition comprises water in a base:water ratio of 1:5 to 1:10.

5. The COMPOSITION, according to claim 4, **characterized in that** said composition comprises water in a base:water ratio of about 1:8.

6. The COMPOSITION, according to any one of claims 1 to 5, **characterized in that** said composition is an emulsion, tablet or soap, liquid, solid or semisolid composition.

7. USE of the composition as defined in any one of claims 1 to 3, **characterized in that** it is in the preparation of a ready-to-use diluted cosmetic composition.

8. A METHOD FOR PREPARING A DILUTED COSMETIC COMPOSITION **characterized in that** it comprises diluting a concentrated cosmetic composition as defined in any one of claims 1 to 3 in base:water ratio of 1:5 to 1:10 under manual shaking or in a mixer.

9. The METHOD according to claim 8, **characterized in that** it uses water in a base:water ratio of about 1:8.
